# EUROPEAN PATENT APPLICATION

(11) **EP 4 470 586 A1**
(43) Date of publication of application: **04.12.2024**
(21) Application number: 24177321.7
(22) Date of filing: 22.05.2024
(51) Int. Cl.: A61M 5/38

(54) **INTRAVENOUS FILTER AIR VENT MEDIA PROTECTION AND ENHANCEMENT**

(30) Priority: 02.06.2023 US 202363505762 P; 15.05.2024 US 202418664458
(71) Applicant: Illinois Tool Works Inc., Glenview IL 60025 (US)
(72) Inventor: LURVEY, Kent Lane, Glenview, 60025 (US); ABBOTT, Mark, Glenview, 60025 (US); BALASUBRAMANIAN, Vinayagasubramani, Glenview, 60025 (US); SIDDHARTHA, Yash, Glenview, 60025 (US)
(74) Representative: HGF

(57) **Abstract**

An intravenous (IV) medication filter device can include a housing having a medication inlet, a medication outlet, and a gas vent. The filter device can include a hydrophobic gas filter inside the housing between the inlet and the gas vent and between the gas vent and the outlet. The hydrophobic gas filter can prevent medication received into the housing via the inlet from existing the housing via the gas vent. The hydrophobic gas filter can permit gas in the housing to exit the housing through the hydrophobic gas filter via the gas vent. The vent hole can have a shape that prevents sterilizing radiation directed at the gas vent from reaching the hydrophobic gas filter. Optionally, a porous plug may be inserted into the gas vent and/or a radiation shroud may be positioned over the gas vent to protect the gas vent from being damaged by the sterilizing radiation.

## Description

### CROSS-REFERENCE TO RELATED APPLICATIONS

This application claims priority to U.S. Provisional Application No. 63/505,782 (filed 02-June-2023), the entire disclosure of which is incorporated herein by reference.

### BACKGROUND

### Technical Field.

The subject matter described herein relates to intravenous (IV) filter devices.

### Discussion of Art.

Some known membrane-type IV filter devices can remove impurities from liquids or fluids that are to be intravenously introduced into the human body. Some filter devices may use carrier fluids that act as carriers for medications, such as saline solutions and nutrient solutions as carrier fluids.

One problem that can be encountered with some known membrane-type IV filter devices is the presence of gas. The carrier fluid may need to be filtered before entering the patient to remove the gas and contaminants in the fluid. Gas in IV filter device or that is mixed with the carrier liquid may prevent effective filtration of the carrier liquid. When the IV filter device is attached to the patient and fluid flow is initiated, gas can enter the conduits connected to the IV filter device or may already be present in the IV filter device. This can be dangerous in that it can pose a significant health risk to the patient.

Some IV filter devices can make use of hydrophobic membrane air vents which are capable of passing gas out of the housings of the IV filter devices through vents while preventing carrier liquids and medications from passing through the membranes (and out of the IV filter devices). However, these hydrophobic membranes can be compromised by sterilization methods used in manufacturing of the IV filter devices. For example, some sterilization methods use radiation or e-beams that can degrade the membrane material properties. This, in turn, can allow the membrane air vent to be degraded and wetted, causing a fluid leak from the IV filter device. This also can pose a safety and health risk to the patient.

It may be desirable to protect the membrane material during sterilization and/or have manufacturing processes that differs from those that are currently available.

### BRIEF DESCRIPTION

An IV medication filter device described herein can include a housing defining an interior chamber. The housing has an inlet through which a liquid medication is received, an outlet through which the liquid medication exits the housing, and a vent hole through which gases exit from the interior chamber. The filter device also can have a hydrophilic filtration media disposed inside the interior chamber of the housing. The hydrophilic filtration media can filter the liquid medication between the inlet and the outlet. The filter device also can include a hydrophobic gas filter disposed inside or outside the housing at the vent hole. The hydrophobic gas filter can prevent entry of contaminants into the interior chamber via the vent hole. The vent hole can be shaped to block e-beam radiation from reaching the hydrophobic gas filter or attenuate the e-beam radiation directed toward the hydrophobic gas filter.

Another IV medication filter device may include a housing defining an interior chamber. The housing can have an inlet through which a liquid medication is received, an outlet through which the liquid medication exits the housing, and a vent hole through which gases exit from the interior chamber. The filter device may have a hydrophilic filtration media disposed inside the interior chamber of the housing. The hydrophilic filtration media can filter the liquid medication between the inlet and the outlet. The filter device can include a hydrophobic gas filter disposed inside or outside the housing at the vent hole. The hydrophobic gas filter can prevent entry of contaminants into the interior chamber via the vent hole. The filter device can include a shroud positioned over the vent hole outside of the housing, the shroud blocking e-beam radiation from reaching the hydrophobic gas filter or attenuating the e-beam radiation directed toward the hydrophobic gas filter.

Another IV medication filter device can include a housing having a medication inlet, a medication outlet, and a gas vent. The filter device also can include a hydrophobic gas filter inside the housing between the inlet and the gas vent and between the gas vent and the outlet. The hydrophobic gas filter can prevent medication received into the housing via the inlet from existing the housing via the gas vent. The hydrophobic gas filter can permit gas in the housing to exit the housing through the hydrophobic gas filter and out of the gas vent. The vent hole can have a shape that prevents sterilizing radiation directed at the gas vent from reaching the hydrophobic gas filter.

### BRIEF DESCRIPTION OF THE DRAWINGS

The inventive subject matter may be understood from reading the following description of non-limiting embodiments, with reference to the attached drawings, wherein below:
Figure 1 illustrates a front view of one example of an IV medication filter device;
Figure 2 illustrates a cross-sectional perspective view of the filter device shown in Figure 1;
Figure 3 illustrates an exploded view of the filter device shown in Figure 1;
Figure 4 illustrates a perspective view of another example of an IV medication filter device;
Figure 5 illustrates a cross-sectional view of one example of a vent hole that can be provided on the filter device of Figure 1 or Figure 4;
Figure 6 illustrates a cross-sectional view of another example of a vent hole that can be provided on the filter device of Figure 1 or Figure 4;
Figure 7 illustrates a cross-sectional view of another example of a vent hole that can be provided on the filter device of Figure 1 or Figure 4;
Figure 8 illustrates a perspective exploded view of one example of an air vent shroud;
Figure 9 illustrates a perspective exploded view of one example of an air vent plug;
Figure 10 illustrates a perspective view of one example of a filter housing having a reinforced air vent;
Figure 11A illustrates a perspective view of one example of a filter housing having an air vent cover that can be located on an exterior surface of the filter;
Figure 11B illustrates a perspective view of another example of a filter housing having an air vent cover that can be located on an interior surface of the filter;
Figure 12 illustrates a cross-sectional view of one example of a filter device having an integral air vent shroud;
Figure 13 illustrates an example arrangement of an array of filters in a sterilization device; and
Figure 14 illustrates a flow chart depicting one example of a method of sterilizing several filters.

### DETAILED DESCRIPTION

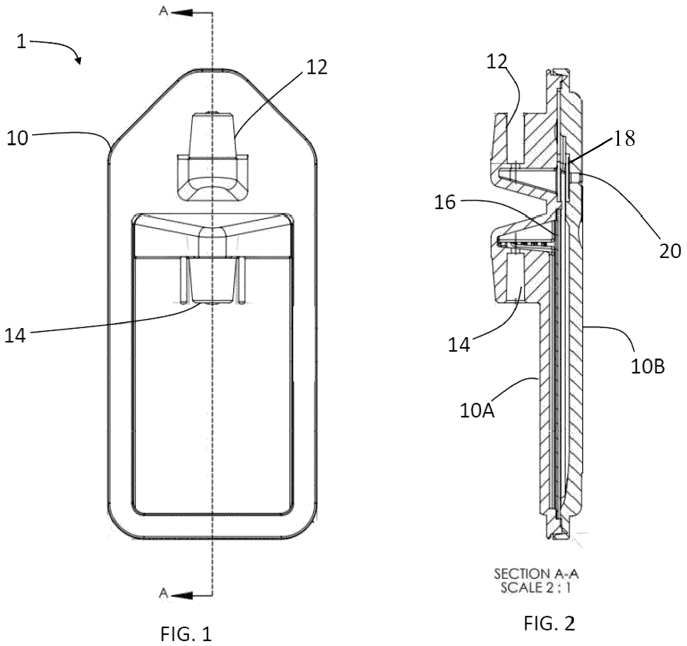

The subject matter relates broadly to membrane-type filter devices, and especially to IV medication filter devices used to remove impurities from liquids or fluids that are to be introduced intravenously to the human body for infusion therapies. Figures 1 through 3 show an example filter device 1 for filtering IV fluid. The filter device 1 can have a generally or predominantly flat, vented housing 10. Alternatively, this housing 10 may have another shape. The housing 10 can include a fluid inlet connector or port 12 and a fluid outlet connector or port 14. The fluid inlet connector 12 and the fluid outlet connector 14 may be located on the same side of the housing 10. Optionally, the connectors 12, 14 may be on different (e.g., opposite) sides of the housing 10. The fluid inlet connector 12 may be elongated or oriented in a first direction with respect to the housing 10. In one example, the fluid inlet connector 12 can be located and aligned along a central axis of the housing 10. The fluid outlet connector 14 may be oriented in a second direction with respect to the housing 10. In one example, the fluid outlet connector 14 can be located and aligned along a central axis of the housing 10 but in an opposite direction as the fluid inlet connector 12.

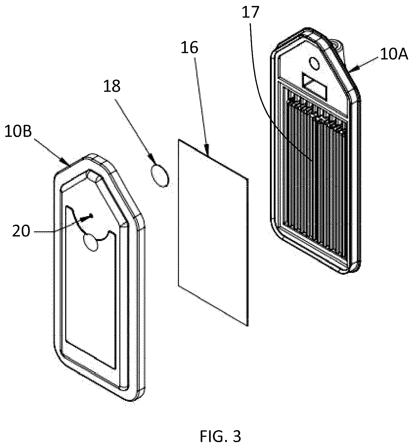

The filter device 1 can be a multi-part construction having a first housing portion 10A and a second housing portion 10B separated by a liquid filter 16 (FIG. 3). Optionally, the housing 10 may be a single continuous body. The liquid filter 16 may be a filtration media, such as a hydrophilic material filter. In one example, the first housing 10A and the second housing 10B can be coupled together on an outer periphery to thereby form a seal for the housing 10. In one example, the first housing 10A and the second housing 10B can be mechanically coupled by interlocking features to snap the bodies together. In one example, the first housing 10A and the second housing 10B can be glued, ultrasonically welded, and/or heat bonded together.

The housing 10 can have an interior chamber once assembled. The liquid filter 16 and a gas filter 18 (FIG. 3) can be positioned between the first housing 10A and the second housing 10B. In one example, the gas filter 18 can be a hydrophobic membrane that allows gas to pass through while generally preventing fluid flow through the material of the filter 18. For description purposes, the gas filter 18 may be referred to as the hydrophobic membrane 18.

In one embodiment, the liquid filter 16 can be generally planar, and secured within the housing 10. Optionally, the liquid filter 16 can have other shapes or textures such as an accordion or folded mesh pattern. The liquid filter 16 can separate the fluid inlet connector 12 and the fluid outlet connector 14 within the housing 10. For example, the liquid filter 16 may be between the fluid inlet connector 12 and the fluid outlet connector 14. Fluid traveling into the fluid inlet connector 12 can pass through the liquid filter 16 before passing out of the filter device 1 through the fluid outlet connector 14.

The filter device 1 can have a vent hole or passage 20 for releasing entrapped gas from the inner chamber of the housing 10. The vent hole 20 can be configured as a passage traversing between the interior chamber of the housing 10 and the exterior of the housing 10. In one example, the vent hole 20 may be arranged orthogonally between the interior surface and the exterior surface of the housing. Gases inside the housing 10 can exit or vent from the housing 10 via the vent hole 20.

In one example, the vent hole 20 can extend one or more directions that are non-orthogonal to the inner surface and the exterior surface of the housing 10. The vent hole 20 can extend along one or more non-orthogonal directions. For example, the vent hole 20 can have a single center axis that oriented at an acute angle or obtuse angle to the inner surface and/or the exterior surface of the housing 10. As another example, the vent hole 20 can have different segments with different center axes. At least one of these axes can be oriented at an acute or obtuse angle to the inner surface and/or the exterior surface of the housing 10. As another example, these center axes of the different segments can be oriented at an acute or obtuse angle to each other (e.g., to the center axis of the next segment). As another example, the different segments of the vent hole can be curved at different radii. As another example, the different segments of the vent hole can be curved at the same or different radii, but with at least two of the segments curved in opposite or different directions. As another example, the segments may have a cross-section that is circular, oval, square, rectangular, triangular, or other shape.

In one embodiment, the hydrophobic membrane 18 can be arranged in proximity to the vent hole 20. In one example, the hydrophobic membrane 18 can be a generally circular or oval shape having a diameter substantially larger than the diameter of the vent hole 20. The hydrophobic membrane 18 can be sized to cover the vent hole 20. The hydrophobic membrane 18 may be positioned abutted to one end of the vent hole 20. For example, the hydrophobic membrane 18 can be positioned on the interior chamber of the housing 10 and couple to an interior opening of the vent hole 20. In one example, the hydrophobic membrane 18 can be positioned on an exterior surface of the housing 10 and couple to an exterior opening of the vent hole 20.

In one example, one of the first or second housing 10A, 10B can have an array of fluid channels 17 formed on and/or in the body of the housing to facilitate a fluid path from the fluid inlet connector 12 to the fluid outlet connector 14 through the liquid filter 16. The fluid outlet connector 14 can typically be coupled to a conduit or other component supplying a liquid (e.g., a medication or other liquid substance) to a patient. In one example, the housing 10 can be generally rectangular.

In one embodiment, fluid can enter the fluid inlet connector 12. This fluid can be directed toward the vent hole 20 by a directed fluid path. Entrapped gas bubbles in the fluid entering the filter device 1 can contact the hydrophobic membrane 18. This gas filter 18 can prevent the gas from remaining in the liquid, venting air or gas to the outside of the filter device 1, while allowing the liquid to pass through the filter device 1. The removed gas can exit the housing via the vent hole 20 while the liquid fluid remains in the housing 10. The fluid can travel within the filter device 1 to the liquid filter 16 and pass there through. The liquid filter 16 can remove particulate, bacteria, and other contaminants from the liquid as the liquid passes through the liquid filter 16. The filter device 1 is shown having a single vent 20, however the filter device 1 may be provided with additional vent holes and respective hydrophobic membranes.

In operation, a liquid medication is received into the inlet 12, such as via a conduit, line, tube, or connector that mates with or connects with the inlet 12. This medication flows inside the housing 10, is internally filtered by the filter 16 to remove contaminants from the medication, and flows to and exits from the housing 10 via the outlet 14. The filter 18 is between the inlet 12 and the vent hole 20 and between the vent hole 20 and the outlet 14. The outlet 14 may be connected to a line, conduit, tube, or other connector that is also connected with a patient to administer the medication to the patient. Air or other gases may enter into the housing via the inlet 12. The air or other gases may be entrained within the medication and flow by the filter 18. The filter 18 can prevent the liquid medication from passing through the filter 18 (and out of the housing 10 via the vent hole). The filter 18 allows the air or other gases in the liquid medication to pass through the filter 18 such that the air or other gases can pass through the filter 18 and exit from the housing 10 via the vent hole 20.

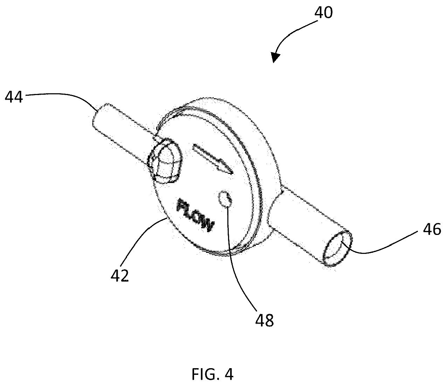

Referring now to Figure 4, in one example, a filter 40 can be configured to operate in substantially the same way as the filter device 1. The filter 40 can be provided with a generally cylindrical housing 42. The housing 42 may be a two-part construction similar to the filter device 1 and can be provided with a hydrophilic filtration media and a hydrophobic vent media arranged within the interior of the housing 42. The filter 40 can be provided with a fluid inlet connector 44 and a fluid outlet connector 46. The filter 40 can have at least one vent hole 48 formed on the housing 42 in proximity to the hydrophobic vent media.

The exemplary filters illustrated in Figures 1-4 may be used to administer medication and fluids intravenously in human patients that can be lifesaving therapies. As such, these medical devices may undergo rigorous sterilization during manufacturing to prevent microbial contamination of the administered fluids. For example, a manufacturing process can include an end-of-line sterilization process where single or multiple filters may pass through to decontaminate and/or sterilize the filters. The term "sterilization" refers to rendering a pathogen substance incapable of reproduction, metabolism and/or growth. While this may often be taken to mean total absence of living organisms, the term may be used herein to refer to a substance free from living organisms to a degree previously agreed or determined to be acceptable. Unless otherwise indicated, the term sterilization may be used herein to also refer to methods and procedures less rigorous than sterilization, for example, disinfection, sanitization, and the like.

One sterilization process with which the present subject matter may be used can include sterilization processes wherein the sterilization medium or sterilant may include steam, dry heat, radiation, plasma, as well as one or more gaseous sterilants, ethylene oxide, one or more liquid sterilants, and the like. The radiation-based processes used may include an electron beam or any electromagnetic spectra including ionizing radiation, pulsed white or ultraviolet light, microwave, and the like. The radiation may comprise gamma or beta radiation. The irradiation may be by, for example, electron beam (e-beam), gamma, X-ray, or other energy forms such as UV radiation. For e-beam radiation, the irradiation dosage may be, for example, from approximately 10 kGy to approximately 50 kGy. Devices and methods of the present subject matter may be described with respect to an e-beam sterilization process wherein a beam of highly charged stream of accelerated electrons may be directed towards a product/material being sterilized. The energy from the e-beam can be absorbed in the material of the product, which can alter various chemical bonds to damage DNA or destroy the reproductive capability of microorganisms present on the product. The e-beam energy can be focused to scan a defined size sometimes in a sweeping motion to create an aligned array of electrons, sometimes referred to herein as an e-beam array. The product being sterilized can be conveyed through the e-beam array at a tightly controlled and measured speed. The sterilization process can take place behind a radiation shield that can be a large, insulated structure, which prevents radiation from leaving the confines of the radiation shield.

In one embodiment, the hydrophobic membrane can be made of or include a thermoplastic material, for example, polyvinylidene fluoride (PVDF) or polytetrafluoroethylene (PTFE). The use of these material for the hydrophobic membrane, sometimes referred to herein as an air vent membrane, can enable use of the filter device 1 for administration of many chemotherapeutic solutions that are known to degrade or cause unwanted wetting or leaking of air vent membranes. For example, membranes made of PVDF or PTFE can be used to administer a variety of IV solutions and medications, including more aggressive solutions that are low in surface tension, or caustic and wet out or degrade the hydrophobic membrane, causing it to leak.

When PVDF materials are exposed to e-beam sterilization, for example, the material properties associated with hydrophobic performance can be impacted. Therefore, embodiments of the present inventive subject matter relate to protecting hydrophobic properties of the air vent membrane upon end-of-line sterilization of the filter device 1 and/or the filter 40.

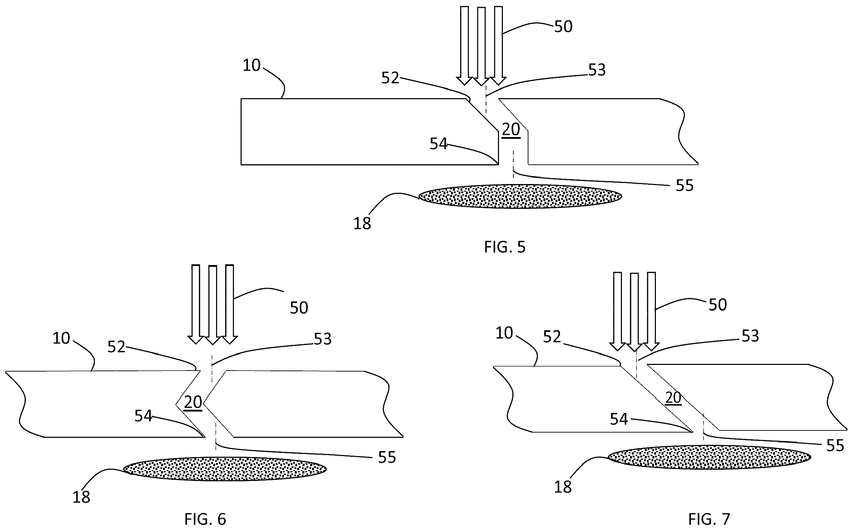

Figures 5-7 illustrate cross-sectional views of exemplary air vent holes 20 that can be formed in filter device 1 and/or filter 40. The vent hole 20 can extend along one or more directions that are non-orthogonal to an inner surface and/or an exterior surface of the housing. In one example, an array of e-beams 50 are depicted schematically as substantially linear unidirectional rays. In practice, e-beam rays may impinge the filter device 1 at an orientation depending on the position of the filter device 1 with respect to the e-beam source. The hydrophobic membrane 18 is shown schematically in Figures 5-7 on the interior of the housing 10 and the e-beams 50 impinge the exterior of the housing 10.

In one example depicted in Figure 5, a vent hole 20 has an exterior opening 52 formed on the exterior of the housing 10 and an interior opening 54 formed on the interior of the housing 10. The exterior opening 52 may be located on the exterior of the housing 10 at a location depicted by a centerline 53. The interior opening 54 may be located on the interior of the housing 10 at a location depicted by a centerline 55. The centerline 55 can be offset from the centerline 53 along the body of the housing 10 when viewed in the plane of the page of Figure 5. The exterior opening 52 and the interior opening 54 can be configured to form a channel through the body of the housing 10 that allows gas to exit the filter. During sterilization, direct exposure of e-beams on the hydrophobic membrane 18 can be attenuated by passing through the material of the housing 10. By offsetting the centerline 53 with respect to the centerline 55, the e-beams may impinge the housing 10 instead of directly impinging the hydrophobic membrane 18. Forming the vent hole 20 so that the exterior opening 52 and the interior opening 54 are non-orthogonal across the body of the housing 10 enables the housing 10 to act as an attenuating component for the hydrophobic membrane 18.

Figures 6 and 7 depict exemplary locations of the exterior opening 52 with respect to the interior opening 54. The vent hole 20 formed by the channel between the exterior opening 52 and the interior opening 54 can be linear, curved, or a combination thereof. The channel can be configured such that the housing 10 provides a physical barrier between the e-beam array and the hydrophobic membrane 18. This physical barrier can be provided by the channel having a certain aspect ratio. In some examples, the length of the vent hole 20 with respect to a width of the vent hole 20 can have an aspect ratio in the range of 1 to 2 as an example. In one embodiment, the vent hole 10 may have an aspect ratio less than or equal to 1 to limit the exposure to the air vent membrane. In one embodiment, the aspect ratio may be less than or equal to 0.5. The vent hole 20 can have different segments that can be oriented at different angles relative to each other, such as depicted in Figure 6. In some examples, the vent hole 20 can be oriented acutely relative to the inner surface and the exterior surface of the housing.

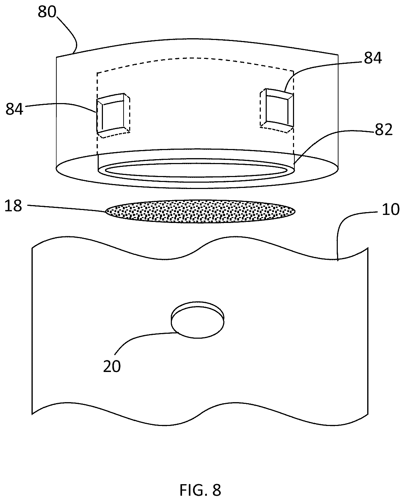

Figure 8 illustrates an air vent shroud 80 that can be coupled to the housing 10 in proximity to the vent hole 20. In one example, the air vent shroud 80 can be configured to couple the hydrophobic membrane 18 to the housing 10. The air vent shroud 80 can be a substantially hollow cylindrical body having a protrusion 82 sized to retain the hydrophobic membrane 18. The air vent shroud 80 may have a closed top surface that blocks or attenuates e-beam or other radiation directed toward the filter 18. The air vent shroud 80 can be provided with a number of channels 84 arrayed radially about the cylindrical body. The channels 84 can be side openings or vents configured to fluidly connect the interior of the cylindrical body to the exterior. The channels 84 can be side air vents. In other examples, the air vent shroud 80 may take other forms such as square, rectilinear, or other shapes. The air vent shroud 80 can be made of similar material as the housing 10. The air vent shroud 80 can be coupled to the housing 10 through ultrasonic welding or other fastening means. The air vent shroud 80 can be positioned to substantially surround the hydrophobic membrane 18 to thereby attenuate direct impact from e-beam sterilization, while still allowing gases to vent or exit the housing 10. In one example, the air vent shroud 80 may be made of a material that is denser than the housing 10. The air vent shroud 80 may be made of a material having different attenuating properties than the material of the housing 10.

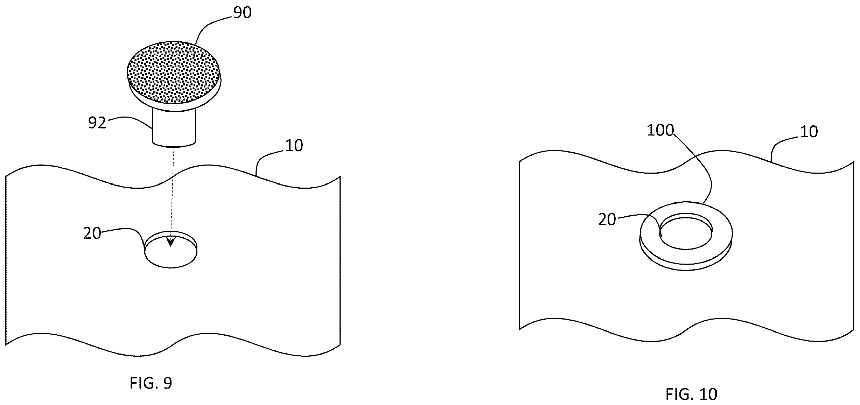

Figure 9 illustrates an air vent plug 90 that can be coupled to the housing 10 on the vent hole 20. In one example, the hydrophobic membrane 18 can be located on the interior of the housing 10 and the air vent plug 90 can be located substantially on the exterior of the housing 10. The air vent plug 90 can have a porous construction to thereby allow passage of air from the vent hole 20 to the atmosphere. In one example, the air vent plug 90 can be made of porous sintered metal, ceramic, plastic, or PTFE, or other material. Porous materials can have a number of pores or cavities arranged in random pattern throughout the material like a sponge. The pores or cavities can be fluidly connected to allow passage there between of gas such as air. The structure of porous materials can be appropriately sized and used as a shield to the e-beam stream. The air vent plug 90 can be provided with a protrusion 92 sized to couple the air vent plug 90 to the vent hole 20 with an interference fit or other fastening means. In one example, the protrusion 92 can be hollow. The protrusion 92 can be made of the same porous material as the air vent plug 90. In one example, the air vent plug 90 can be substantially cylindrical and have a diameter sized to cover the air vent hole 20. Porous materials may have a high shielding capability to attenuate gamma, x-ray, and/or e-beams. During sterilization, impinging e-beams are attenuated or blocked from directly impacting the hydrophobic membrane by the porous air vent plug 90. For example, the pores may be arranged such that there is no single straight line path through the plug 90 for any e-beam to pass and strike the membrane.

Figure 10 illustrates a reinforced air vent 100 that can be provided on the housing 10. In one example, the reinforced air vent 100 can an annular protrusion surrounding the vent hole 20. The reinforced air vent 100 can take many forms and may be considered any thickening of housing material surrounding the vent hole 20. In some embodiments, the reinforced air vent 100 can have a different material than the housing 10. For example, the reinforced air vent 100 can be made of a UV or electromagnetic irradiation energy or e-beam attenuating or blocking material. In some examples, the material forming the reinforced air vent 100 can be an additive to resin forming the housing 10. In one example, the material forming the reinforced air vent 100 can have a higher density than the material forming the housing 10. In one example, the reinforced air vent 100 can be an annular cylindrical ring. In some examples, the reinformed air vent 100 can have a conical or convex shape. The reinforced air vent 100 can lengthen the vent hole 20. In one example, the reinforced air vent 100 can be used to change the aspect ratio of the vent hole 20.

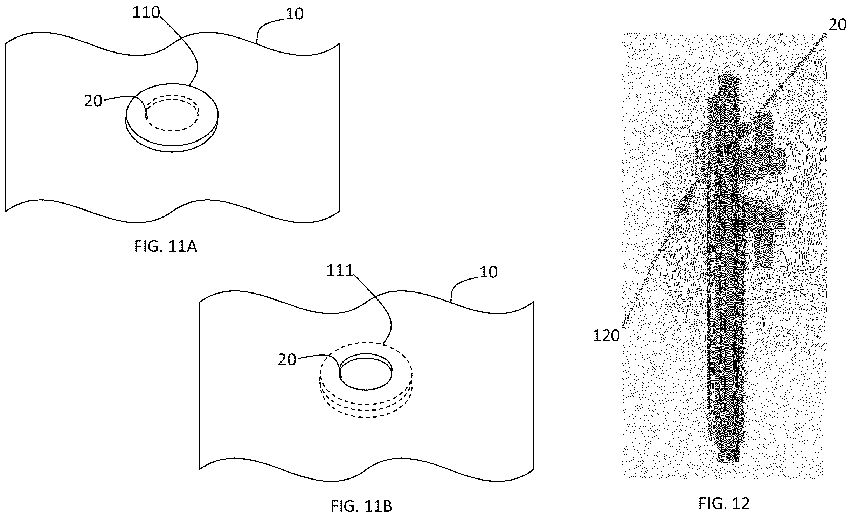

Figure 11A illustrates an air vent cover 110 that can be adhered to the exterior of the housing 10 to thereby cover the vent hole 20. In one example, the air vent cover can be a dark sticker whereby the sticker acts as a diffuser, attenuator, or buffer from irradiation or e-beam streams that can impact the hydrophobic membrane 18. In one example, the dark sticker can be black, blue, brown, or other color darker than pastel for use in light or energy absorption or disruption. In some examples, the air vent cover 110 can be made of PA monofilament mesh, PA membrane, or PET knitted fabric, among others. In one example, the air vent cover 110 can be made of a material that changes color based on the dosage of exposure to the irradiation or e-beam source. The change in color can provide a visual indication of the exposure dose of the filter during sterilization. The air vent cover 110 can be made of a flexible material having an adhesive on one side, or it can be attached by heat staking or ultrasonic welding. In one example, the air vent cover 110 can be removed from the filter device 1 before use.

Figure 11B illustrates an air vent cover 111 that can be adhered to the interior of the housing 10 to thereby cover the vent hole 20. In one example, the air vent cover 11 1 can adhere to the housing 10 on an interior surface to cover the vent hole 20. In some examples, one or more air vent covers 111 can be used to form laminated layers configured to adhere to the hydrophobic membrane 18 or to the housing 10. In some examples, additional laminated layers of the same hydrophobic membrane material can be used to double (or more) the thickness of the hydrophobic membrane 18. In other examples, the air vent cover 111 can be a laminated media bonded to the hydrophobic membrane 18 and located on the interior of the housing 10 in proximity to the vent hole 20. The laminated layers can be made of irradiation or e-beam diffusing or blocking material, for example, polyamide (PA) monofilament mesh, a PA membrane, or polyethylene terephthalate knitted fabric, or other such membrane as may be able to resist the irradiation or e-beam dose. The laminated layers can be PET and/or other porous media adhered to the PVDF or PTFE material of the hydrophobic membrane 18. In some examples, the laminated layers are made of PVDF or PTFE. The laminated layers can be positioned to between the hydrophobic membrane 18 and any irradiation or impinging e-beam stream.

Figure 12 shows a cross-sectional view of the filter device 1 provided with an integral air vent shroud 120 arranged in proximity to the vent hole 20. The integral air vent shroud 120 can be formed integral with the housing 10, or it may be integrally molded, snap fit attached, ultrasonically welded, glued, or otherwise affixed to the housing 10. In one example, the integral air vent shroud 120 can be a protrusion having a uniform cross-section and extends over the vent hole 20 in such a way that there can be an opening between the exit of the vent hole and the integral air vent shroud 120. The integral air vent shroud 120 can be an attenuating segment of material arranged to block the line of sight of e-beam stream or irradiation directed towards the hydrophobic membrane 18. In one example, the cross-sectional thickness of the integral air vent shroud 120 can be greater than the thickness of the first housing 10A, the second housing 10B, and/or the assembly of the housing 10. In one example, the integral air vent shroud 120 may be formed with resin having attenuating additives that reduce and/or buffer the e-beam or irradiation. The integral air vent shroud 120 shown in cross-section in Figure 12 may take a concave or convex shape with respect to the housing 10. In other examples, the air vent shroud 120 may have a non-uniform cross-section.

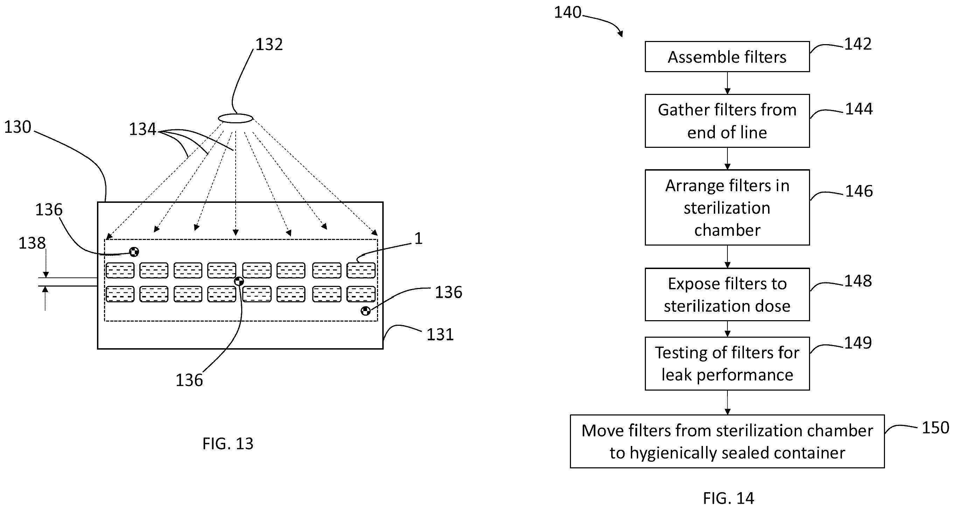

Turning now to Figure 13, a method for e-beam sterilizing a number of filters 1 and/or filters 40 will be described. An e-beam sterilization device 130 can be provided having a table 131 located in a path of an e-beam emitter 132 perpendicularly positioned above the table. The e-beam emitter 132 can emit an e-beam array 134. The e-beam emitter 132 can be arranged in a central location within the e-beam sterilization device 130. The e-beam sterilization device 130 can include a housing or structure configured to form a chamber used to substantially enclose the table 131, the e-beam emitter 132, and the e-beam array 134. Exposure from the e-beam array 134 can provide a more intense exposure or dose if the e-beam hits the air vent membrane, or hydrophobic membrane 18, directly, or in a line of sight, on any object being sterilized. The e-beam array 134 may be generally linear with respect to the table 131.

For discussion purposes, several filters 1 will be used as an illustrative example. In one example, the filters 1 can be arranged end-to-end in two rows on the table 131. The rows can be separated by a distance generally between 5-6 inches and can be dependent on the size and shape of the filter device 1. When the filter device 1 is arranged closer to the center of the of the e-beam array 134 as opposed to the peripheral e-beam array 134, the e-beam exposure can be more consistent in its dose range, and potentially lower in dose, as compared to the level of exposure experienced when the filter device 1 is located further away or at a more peripheral location from the e-beam emitter 132. Thus, sterilization can be delivered in a way that minimizes the e-beam dose range and subsequent intensity, thereby limiting the potential for hydrophobic membrane degradation from an inadvertently higher dose.

The illustrative embodiments of attenuating methods and structures presented herein with reference to Figures 1-12 can be used to generally diminish the dose or intensity of the irradiation or e-beam stream in the general location of the air vent membrane 18. A minimum sterilizing (or pathogen killing) e-beam dose can still insure the kill of pathogens based on readings from dosimeters 136 located in and or around the filters 1 within the e-beam sterilization device 130, thus a minimum sterilizing dose can be accomplished. Generally, e-beam irradiation can be similar to gamma irradiation, in that both penetrate the mass of the object and make a kill of pathogens throughout the object.

During sterilization, the intensity of the e-beam array 134 can be controlled in multiple ways. By arranging the filters 1 in rows as depicted and described with reference to Figure 13, the intensity or dose of the delivered e-beam array 134 can be delivered at a value lower than an intensity known to degrade the hydrophobic membrane 18. For example, when the filters 1 are placed in the sterilization device 130 in no particular arrangement, orientation, or in boxes or bags with respect to the e-beam emitter 132, the intensity of the e-beam array 134 may need to be set at a higher intensity range in order to provide the minimum acceptable dosage to filters at all distal locations from the e-beam emitter 132. Arranging the filters 1 in a pattern having two rows aligned and centrally located with the e-beam array 132 can provide a more uniform exposure range across the filters 1, thereby allowing the ability to reduce the overall exposure dose. The more uniform exposure may therefore enable a lower effective intensity to be delivered.

Figure 14 shows a flow chart depicting a method 140 generally described in reference to Figure 13. In one example, the method 140 can begin at a step 142 where filters such as the filters 1 or the filters 40 can be assembled in a manufacturing facility. The method 140 can proceed to a step 144 where the filters can be gathered from the end of the assembly line by a human, a robot, a conveyor mechanism, or other means to ready the filters for the next step. The method 140 can proceed to a step 146 where the filters can be arranged in a sterilization chamber. In one example, the filters can be arranged end to end in two rows to present the filters to the e-beam emitter 132, for example. The method 140 can proceed to a step 148 where the sterilization dose can be administered, for example, by emitting the e-beam array 134. The method 140 can proceed to a step 149 where the filters may be used for testing purposes to show that the lower e-beam dose does not degrade the air vent membrane to a point where the air vent or the filter does not meet the appropriate leak performance requirements. The method 140 can proceed to a step 150 where the sterilized filters can be removed from the sterilization chamber and packaged as bulk sterile filters, to be later fully assembled in a hygienic method to the larger IV administration device. In one example, the filters may be placed in hygienically sealed containers to prevent contamination. In another example, the full and complete IV administration device may be placed in the same sterilization chamber as the e-beam array 134.

An IV medication filter device described herein can include a housing defining an interior chamber. The housing has an inlet through which a liquid medication is received, an outlet through which the liquid medication exits the housing, and a vent hole through which gases exit from the interior chamber. The filter device also can have a hydrophilic filtration media disposed inside the interior chamber of the housing. The hydrophilic filtration media can filter the liquid medication between the inlet and the outlet. The filter device also can include a hydrophobic gas filter disposed inside or outside the housing at the vent hole. The hydrophobic gas filter can prevent entry of contaminants into the interior chamber via the vent hole. The vent hole can be shaped to block e-beam radiation from reaching the hydrophobic gas filter or attenuate the e-beam radiation directed toward the hydrophobic gas filter.

The vent hole may not encompass any linear path that intersects the hydrophobic gas filter but does not extend through the housing. The vent hole can be an elongated conduit oriented at an acute angle to the hydrophobic gas filter. The vent hole can be segmented into several linear conduits oriented at an acute angle to each other.

The filter device optionally may include a shroud positioned over the vent hole outside of the housing. The shroud can block the e-beam radiation from reaching the hydrophobic gas filter or attenuating the e-beam radiation directed toward the hydrophobic gas filter. The shroud may have side openings through which the gas vented out of the housing via the vent hole passes.

The IV filter also may include a gas permeable plug disposed in the vent hole. The gas permeable plug can be porous for the gas to pass through as the gas exits the housing via the vent hole while also attenuating the e-beam radiation directed toward the hydrophobic gas filter. The housing can have an increased thickness around a perimeter of the vent hole relative to a remainder of the housing.

Another IV medication filter device may include a housing defining an interior chamber. The housing can have an inlet through which a liquid medication is received, an outlet through which the liquid medication exits the housing, and a vent hole through which gases exit from the interior chamber. The filter device may have a hydrophilic filtration media disposed inside the interior chamber of the housing. The hydrophilic filtration media can filter the liquid medication between the inlet and the outlet. The filter device can include a hydrophobic gas filter disposed inside or outside the housing at the vent hole. The hydrophobic gas filter can prevent entry of contaminants into the interior chamber via the vent hole. The filter device can include a shroud positioned over the vent hole outside of the housing, the shroud blocking e-beam radiation from reaching the hydrophobic gas filter or attenuating the e-beam radiation directed toward the hydrophobic gas filter.

The shroud can have side openings through which the gas vented out of the housing via the vent hole passes. The vent hole can be shaped to block the e-beam radiation from reaching the hydrophobic gas filter or attenuate the e-beam radiation directed toward the hydrophobic gas filter. The vent hole may not encompass any linear path that intersects the hydrophobic gas filter but does not extend through the housing. The vent hole can be an elongated conduit oriented at an acute angle to the hydrophobic gas filter. The vent hole can be segmented into several linear conduits oriented at an acute angle to each other.

The filter device also may include a gas permeable plug disposed in the vent hole. The gas permeable plug can be porous for the gas to pass through as the gas exits the housing via the vent hole while also attenuating the e-beam radiation directed toward the hydrophobic gas filter. The housing can have an increased thickness around a perimeter of the vent hole relative to a remainder of the housing.

Another IV medication filter device can include a housing having a medication inlet, a medication outlet, and a gas vent. The filter device also can include a hydrophobic gas filter inside the housing between the inlet and the gas vent and between the gas vent and the outlet. The hydrophobic gas filter can prevent medication received into the housing via the inlet from existing the housing via the gas vent. The hydrophobic gas filter can permit gas in the housing to exit the housing through the hydrophobic gas filter and out of the gas vent. The vent hole can have a shape that prevents sterilizing radiation directed at the gas vent from reaching the hydrophobic gas filter.

The filter device also can include a radiation shroud coupled to the housing outside of the housing over the gas vent. The radiation shroud can attenuate or block the sterilizing radiation from reaching the hydrophobic gas filter via the gas vent.

Provided herein is an example of an intravenous medication filter for infusion therapy that can include a housing defining an interior chamber, the housing may have an inlet through which a liquid medication can be received, an outlet through which the liquid medication can exit the housing after being filtered, and a vent hole through which gases can exit from the interior chamber. The intravenous medication filter can include a hydrophilic filtration media disposed inside the interior chamber of the housing, the hydrophilic filtration media can be configured to remove one or more constituents from the liquid medication to filter the liquid medication. The intravenous medication filter can include a hydrophobic gas filter disposed inside or outside the housing between the interior chamber of the housing and the vent hole, the hydrophobic gas filter can be configured to prevent entry of contaminants into the interior chamber via the vent hole, wherein the vent hole one or more of: (a) extends along one or more directions that can be non-orthogonal to an inner surface and an exterior surface of the housing or (b) can have an aspect ratio equal to or less than 2.

In one example, the vent hole can include different segments that are oriented at different angles relative to each other. The vent hole can be acutely oriented relative to the inner surface and the exterior surface of the housing. The vent hole extends along a nonlinear path. The vent hole one or more of can (a) extend along the one or more directions or (b) have the aspect ratio equal to or less than 1.

Provided herein is an example of an intravenous medication filter for infusion therapy that can have a housing defining an interior chamber. The housing can have an inlet through which a liquid medication can be received, an outlet through which the liquid medication can exit the housing after being filtered, and a vent hole through which one or more gases can exit from the interior chamber. The intravenous medication filter can include a liquid filter disposed inside the interior chamber of the housing. The liquid filter can be configured to remove one or more constituents from the liquid medication to filter the liquid medication. The intravenous medication filter can include a gas filter disposed inside the housing between the interior chamber of the housing and the vent hole. The gas filter can be configured to allow gas to vent out of the filter and to prevent entry of one or more contaminants into the interior chamber via the vent hole. The intravenous medication filter can include an attenuating component disposed over the vent hole such that the attenuating component reduces a dose of sterilizing e-beam stream or ionizing irradiation directed toward the gas filter.

In one example, the attenuating component can include a shroud disposed over and enclosing the gas filter and the vent hole between the shroud and the housing. The shroud can include one or more vent openings through which the one or more gases exit from the interior chamber via the vent hole and the one or more vent openings in the shroud. In one example, the attenuating component can include a porous plug disposed in the vent hole and including one or more pores through which the one or more gases can exit from the interior chamber via the vent hole. In one example, the attenuating component can include one or more of an adhesive sticker disposed over the vent hole.

Provided herein is an example of an intravenous medication filter for infusion therapy that may include a housing defining an interior chamber, the housing having an inlet through which a liquid medication can be received, an outlet through which the liquid medication can exit the housing after being filtered, and a vent hole through which one or more gases can exit from the interior chamber. The intravenous medication filter can include a liquid filter disposed inside the interior chamber of the housing, the liquid filter can be configured to remove one or more constituents from the liquid medication to filter the liquid medication. The intravenous medication filter can include a gas filter disposed inside the housing between the interior chamber of the housing and the vent hole, the gas filter can be configured to allow gases to exist and prevent entry of one or more contaminants into the interior chamber via the vent hole. The intravenous medication filter can include a dosimeter coupled with the housing proximate to the vent hole and can be configured to change appearance based on a dose of sterilizing e-bean stream or irradiation that is received by the gas filter.

Provided herein is an intravenous medication filter for infusion therapy that can include a housing defining an interior chamber, the housing can have an inlet through which a liquid medication can be received, an outlet through which the liquid medication can exit the housing after being filtered, and a vent hole through which one or more gases can exit from the interior chamber. The intravenous medication filter can include a liquid filter disposed inside the interior chamber of the housing, the liquid filter can be configured to remove one or more constituents from the liquid medication to filter the liquid medication. The intravenous medication filter can include a gas filter disposed inside the housing between the interior chamber of the housing and the vent hole, the gas filter can be configured to allow gases to exit and to prevent entry of one or more contaminants into the interior chamber via the vent hole. The housing can have an attenuating segment proximate to the gas filter that reduces a dose of sterilizing radiation directed toward the gas filter while sterilizing the gas filter.

In one example, the attenuating segment of the housing can have a greater thickness relative to a remainder of the housing. In one example, the attenuating segment of the housing can be formed from a resin containing an attenuating additive that can reduce the dose of the sterilizing e-beam stream and irradiation. In one example, the attenuating segment of the housing can have a greater density relative to a remainder of the housing. In one example, the attenuating segment of the housing can be concave or convex relative to the remainder of the housing. In one example, the attenuating segment that may be concave or convex deflects at least some of the sterilizing e-beam stream or irradiation away from the gas filter or dissipates the at least some of the sterilizing e-beam stream or irradiation before reaching the gas filter.

Provided herein is an example of an intravenous medication filter for infusion therapy that can include a housing defining an interior chamber, the housing can have an inlet through which a liquid medication can be received, an outlet through which the liquid medication can exit the housing after being filtered, and a vent hole through which one or more gases can exit from the interior chamber. In one example, a liquid filter can be disposed inside the interior chamber of the housing, the liquid filter can configured to remove one or more constituents from the liquid medication to filter the liquid medication. The intravenous medication filter can include a gas filter disposed inside the housing between the interior chamber of the housing and the vent hole, the gas filter can be configured to allow gases to vent and prevent entry of one or more contaminants into the interior chamber via the vent hole. The gas filter one or more of can: (a) include or can be formed from one or more layers or laminates of an irradiation or e-beam stream diffusing or blocking material or (b) can be formed from multiple, separate layers of polytetrafluoroethylene (PTFE) or polyvinylidene fluoride (PVDF).

In one example, the gas filter can include or is formed from the one or more gas filter materials, or multiple thickness of a single layer.

Provided herein is a method that can include arranging a plurality of intravenous medication filters for infusion therapy in a pattern within a chamber of an e-beam sterilization device, each filter can have a housing defining interior chambers and having an inlet through which liquid medication can be received, an outlet through which the liquid medication can exit the housing after being filtered, and a vent hole through which one or more gases can exit from the interior chamber. Each filter can include a gas filter disposed inside the housing between the interior chambers and the vent hole, the filters arranged in the pattern such that as they are presented to an e-beam stream in proximity to a center of the e-beam sterilization equipment. The method can include exposing the gas filters to an e-beam to sterilize the filters. The filters can be arranged in the pattern such that the gas filters are no farther than generally 5.8 inches apart.

In one example, the method can include the step of arranging the several intravenous medication filters may include stacking the several intravenous medication filters in two rows separated by a gap having a width of approximately 5.8 inches.

The inventive subject matter described herein can reduce or prevent premature deterioration of the filter media in the housing of an IV medication filter device that otherwise is caused by exposure of the filter media to sterilizing (or other) radiation energy, such as UV or e-beam radiation. In one example, the vent hole through the housing of the filter device may define an internal shape or path that is angled so that the vent hole does not encompass a linear path that extends through or intersects the filter. The path of the vent hole may be linear, but oriented at an acute angle to the housing and the filter such that e-beams or other energy cannot reach the filter without first reaching (and being attenuated or blocked) by the material of the housing. Optionally, the vent hole may define several linear paths that are angled relative to each other while not including or encompassing any linear path from extending through the vent hole and intersecting the filter. The vent hole may be or extend along a linear path that is orthogonal (e.g., perpendicular) to the filter. But the vent hole may have a sufficiently large aspect ratio (defined by the depth or length of the vent hole through the housing divided by the diameter of the vent hole) that few e-beams or other radiation reach the filter, thereby protecting the filter from deterioration from this radiation. For example, the aspect ratio may be at least two, a value found by the inventors to sufficiently protect the filter from e-beam radiation deterioration.

As another example, a shroud may be placed over the vent hole. This shroud may have a closed top surface that blocks or attenuates any e-beam or other radiation directed perpendicularly toward the filter. The shroud may be open along its sides to provide transverse holes that are transversely oriented (e.g., perpendicular) to the vent hole. As another example, a gas permeable plug may be placed into the vent hole. This plug may be porous to allow gas to pass out of the vent hole through the pores in the plug. The plug may be dense enough, however, to attenuate or block e-beam or other radiation directed toward the filter.

The housing may be thicker in an immediate area around the perimeter of the vent hole. This thicker housing may impede, attenuate, or block more e-beam or other radiation directed toward the filter (relative to the housing not being thicker at and around the perimeter of the vent hole).

The singular forms "a", "an", and "the" include plural references unless the context clearly dictates otherwise. "Optional" or "optionally" means that the subsequently described event or circumstance may or may not occur, and that the description may include instances where the event occurs and instances where it does not. Approximating language, as used herein throughout the specification and claims, may be applied to modify any quantitative representation that could permissibly vary without resulting in a change in the basic function to which it may be related. Accordingly, a value modified by a term or terms, such as "about," "substantially," and "approximately," may be not to be limited to the precise value specified. In at least some instances, the approximating language may correspond to the precision of an instrument for measuring the value. Here and throughout the specification and claims, range limitations may be combined and/or interchanged, such ranges may be identified and include all the sub-ranges contained therein unless context or language indicates otherwise.

This written description uses examples to disclose the embodiments, including the best mode, and to enable a person of ordinary skill in the art to practice the embodiments, including making and using any devices or systems and performing any incorporated methods. The claims define the patentable scope of the disclosure, and include other examples that occur to those of ordinary skill in the art. Such other examples are intended to be within the scope of the claims if they have structural elements that do not differ from the literal language of the claims, or if they include equivalent structural elements with insubstantial differences from the literal language of the claims.
Certain embodiments of the invention are described in the following clauses:
Clause 1. An intravenous (IV) medication filter device, the filter device comprising:
   a housing defining an interior chamber, the housing having an inlet through which a liquid medication is received, an outlet through which the liquid medication exits the housing, and a vent hole through which gases exit from the interior chamber;
   a hydrophilic filtration media disposed inside the interior chamber of the housing, the hydrophilic filtration media configured to filter the liquid medication between the inlet and the outlet; and
   a hydrophobic gas filter disposed inside or outside the housing at the vent hole, the hydrophobic gas filter configured to prevent entry of contaminants into the interior chamber via the vent hole,
   wherein the vent hole is shaped to block electron beam (e-beam) radiation from reaching the hydrophobic gas filter or attenuate the e-beam radiation directed toward the hydrophobic gas filter.
Clause 2. The IV medication filter device of clause 1, wherein the vent hole does not encompass any linear path that intersects the hydrophobic gas filter but does not extend through the housing.
Clause 3. The IV medication filter device of clause 1, wherein the vent hole is an elongated conduit oriented at an acute angle to the hydrophobic gas filter.
Clause 4. The IV medication filter device of clause 1, wherein the vent hole is segmented into several linear conduits oriented at an acute angle to each other.
Clause 5. The IV medication filter device of clause 1, further comprising:
   a shroud positioned over the vent hole outside of the housing, the shroud blocking the e-beam radiation from reaching the hydrophobic gas filter or attenuating the e-beam radiation directed toward the hydrophobic gas filter.
Clause 6. The IV medication filter device of clause 5, wherein the shroud has side openings through which the gas vented out of the housing via the vent hole passes.
Clause 7. The IV medication filter device of clause 1, further comprising:
   a gas permeable plug disposed in the vent hole.
Clause 8. The IV medication filter device of clause 7, wherein the gas permeable plug is porous for the gas to pass through as the gas exits the housing via the vent hole while also attenuating the e-beam radiation directed toward the hydrophobic gas filter.
Clause 9. The IV medication filter device of clause 1, wherein the housing has an increased thickness around a perimeter of the vent hole relative to a remainder of the housing.
Clause 10. An intravenous (IV) medication filter device, the filter device comprising:
   a housing defining an interior chamber, the housing having an inlet through which a liquid medication is received, an outlet through which the liquid medication exits the housing, and a vent hole through which gases exit from the interior chamber;
   a hydrophilic filtration media disposed inside the interior chamber of the housing, the hydrophilic filtration media configured to filter the liquid medication between the inlet and the outlet;
   a hydrophobic gas filter disposed inside or outside the housing at the vent hole, the hydrophobic gas filter configured to prevent entry of contaminants into the interior chamber via the vent hole; and
   a shroud positioned over the vent hole outside of the housing, the shroud blocking electron beam (e-beam) radiation from reaching the hydrophobic gas filter or attenuating the e-beam radiation directed toward the hydrophobic gas filter.
Clause 11. The IV medication filter device of clause 10, wherein the shroud has side openings through which the gas vented out of the housing via the vent hole passes.
Clause 12. The IV medication filter device of clause 10, wherein the vent hole is shaped to block the e-beam radiation from reaching the hydrophobic gas filter or attenuate the e-beam radiation directed toward the hydrophobic gas filter.
Clause 13. The IV medication filter device of clause 12, wherein the vent hole does not encompass any linear path that intersects the hydrophobic gas filter but does not extend through the housing.
Clause 14. The IV medication filter device of clause 12, wherein the vent hole is an elongated conduit oriented at an acute angle to the hydrophobic gas filter.
Clause 15. The IV medication filter device of clause 12, wherein the vent hole is segmented into several linear conduits oriented at an acute angle to each other.
Clause 16. The IV medication filter device of clause 10, further comprising:
   a gas permeable plug disposed in the vent hole.
Clause 17. The IV medication filter device of clause 16, wherein the gas permeable plug is porous for the gas to pass through as the gas exits the housing via the vent hole while also attenuating the e-beam radiation directed toward the hydrophobic gas filter.
Clause 18. The IV medication filter device of clause 10, wherein the housing has an increased thickness around a perimeter of the vent hole relative to a remainder of the housing.
Clause 19. An IV medication filter device comprising:
   a housing having a medication inlet, a medication outlet, and a gas vent; and
   a hydrophobic gas filter inside the housing between the inlet and the gas vent and between the gas vent and the outlet, the hydrophobic gas filter configured to prevent medication received into the housing via the inlet from existing the housing via the gas vent, the hydrophobic gas filter configured to permit gas in the housing to exit the housing through the hydrophobic gas filter and out of the gas vent,
   wherein the vent hole has a shape that prevents sterilizing radiation directed at the gas vent from reaching the hydrophobic gas filter.
Clause 20. The IV medication filter device of clause 19, further comprising:
   a radiation shroud coupled to the housing outside of the housing over the gas vent, the radiation shroud configured to attenuate or block the sterilizing radiation from reaching the hydrophobic gas filter via the gas vent.

## Claims

1. An intravenous (IV) medication filter device, the filter device comprising:
a housing defining an interior chamber, the housing having an inlet through which a liquid medication is received, an outlet through which the liquid medication exits the housing, and a vent hole through which gases exit from the interior chamber;
a hydrophilic filtration media disposed inside the interior chamber of the housing, the hydrophilic filtration media configured to filter the liquid medication between the inlet and the outlet; and
a hydrophobic gas filter disposed inside or outside the housing at the vent hole, the hydrophobic gas filter configured to prevent entry of contaminants into the interior chamber via the vent hole,
wherein the vent hole is shaped to block electron beam (e-beam) radiation from reaching the hydrophobic gas filter or attenuate the e-beam radiation directed toward the hydrophobic gas filter.

2. The IV medication filter device of claim 1, wherein the vent hole does not encompass any linear path that intersects the hydrophobic gas filter but does not extend through the housing.

3. The IV medication filter device of claim 1, wherein the vent hole is an elongated conduit oriented at an acute angle to the hydrophobic gas filter, or wherein the vent hole is segmented into several linear conduits oriented at an acute angle to each other.

4. The IV medication filter device of claim 1, further comprising:
a shroud positioned over the vent hole outside of the housing, the shroud blocking the e-beam radiation from reaching the hydrophobic gas filter or attenuating the e-beam radiation directed toward the hydrophobic gas filter, and optionally wherein the shroud has side openings through which the gas vented out of the housing via the vent hole passes.

5. The IV medication filter device of claim 1, further comprising:
a gas permeable plug disposed in the vent hole, and optionally wherein the gas permeable plug is porous for the gas to pass through as the gas exits the housing via the vent hole while also attenuating the e-beam radiation directed toward the hydrophobic gas filter.

6. The IV medication filter device of claim 1, wherein the housing has an increased thickness around a perimeter of the vent hole relative to a remainder of the housing.

7. An intravenous (IV) medication filter device, the filter device comprising:
a housing defining an interior chamber, the housing having an inlet through which a liquid medication is received, an outlet through which the liquid medication exits the housing, and a vent hole through which gases exit from the interior chamber;
a hydrophilic filtration media disposed inside the interior chamber of the housing, the hydrophilic filtration media configured to filter the liquid medication between the inlet and the outlet;
a hydrophobic gas filter disposed inside or outside the housing at the vent hole, the hydrophobic gas filter configured to prevent entry of contaminants into the interior chamber via the vent hole; and
a shroud positioned over the vent hole outside of the housing, the shroud blocking electron beam (e-beam) radiation from reaching the hydrophobic gas filter or attenuating the e-beam radiation directed toward the hydrophobic gas filter.

8. The IV medication filter device of claim 7, wherein the shroud has side openings through which the gas vented out of the housing via the vent hole passes.

9. The IV medication filter device of claim 7, wherein the vent hole is shaped to block the e-beam radiation from reaching the hydrophobic gas filter or attenuate the e-beam radiation directed toward the hydrophobic gas filter.

10. The IV medication filter device of claim 9, wherein the vent hole does not encompass any linear path that intersects the hydrophobic gas filter but does not extend through the housing.

11. The IV medication filter device of claim 9, wherein the vent hole is an elongated conduit oriented at an acute angle to the hydrophobic gas filter, or wherein the vent hole is segmented into several linear conduits oriented at an acute angle to each other.

12. The IV medication filter device of claim 10, further comprising:
a gas permeable plug disposed in the vent hole, and optionally wherein the gas permeable plug is porous for the gas to pass through as the gas exits the housing via the vent hole while also attenuating the e-beam radiation directed toward the hydrophobic gas filter.

13. The IV medication filter device of claim 10, wherein the housing has an increased thickness around a perimeter of the vent hole relative to a remainder of the housing.

14. An IV medication filter device comprising:
a housing having a medication inlet, a medication outlet, and a gas vent; and
a hydrophobic gas filter inside the housing between the inlet and the gas vent and between the gas vent and the outlet, the hydrophobic gas filter configured to prevent medication received into the housing via the inlet from existing the housing via the gas vent, the hydrophobic gas filter configured to permit gas in the housing to exit the housing through the hydrophobic gas filter and out of the gas vent,
wherein the vent hole has a shape that prevents sterilizing radiation directed at the gas vent from reaching the hydrophobic gas filter.

15. The IV medication filter device of claim 14, further comprising:
a radiation shroud coupled to the housing outside of the housing over the gas vent, the radiation shroud configured to attenuate or block the sterilizing radiation from reaching the hydrophobic gas filter via the gas vent.
